# EUROPEAN PATENT APPLICATION

(11) **EP 4 516 099 A1**
(43) Date of publication of application: **05.03.2025**
(21) Application number: 23208858.3
(22) Date of filing: 09.11.2023
(51) Int. Cl.: A01N 25/10, A01N 25/34, A01N 35/06, A01P 1/00, A61P 31/04, B65D 81/26, C08K 3/34, C08K 5/07

(54) **COMPOSITION FOR FOOD CONTAINER AND METHOD FOR MANUFACTURING FOOD CONTAINER USING SAME**

(30) Priority: 01.09.2023 KR 20230115972
(71) Applicant: Lock & Lock Co., Ltd, Anseong-si Gyeonggi-do (KR)
(72) Inventor: Choi, So Jeong, Hwaseong-si, Gyeonggi-do (KR)
(74) Representative: Petraz, Gilberto Luigi

(57) **Abstract**

The present disclosure relates to a composition for a food container and a method for manufacturing a food container using the same, and specifically to a composition for a food container, which includes zeolite, an antibacterial additive, and a porous adsorption powder, thereby adsorbing and removing ethylene gas, carbonic acid gas, and the like generated by a respiratory action of fruit vegetables and fruits, and preventing spoilage caused by microorganisms with an antibacterial action so that it can freshly keep fruit vegetables and fruits for a long period of time, and a method for manufacturing a food container using the same.

## Description

### [Technical Field]

The present disclosure relates to a composition for a food container and a method for manufacturing a food container using the same. Specifically, the present disclosure relates to a composition for a food container, which includes zeolite, an antibacterial additive, and a porous adsorption powder, thereby adsorbing and removing ethylene gas, carbonic acid gas, and the like generated by a respiratory action of fruit vegetables and fruits, and preventing spoilage caused by microorganisms with an antibacterial action so that it can keep fruit vegetables and fruits fresh for a long period of time, and a method for manufacturing a food container using the same.

### [Background Art]

Since fruit vegetables are alive, they continue to respire even after harvest, and the respiratory action means taking in oxygen and releasing carbon dioxide. When fruit vegetables and fruits are stored in airtight containers for a long period of time, they lose their function of an assimilation action and consume stored substances such as starch, organic acids, and sugars through the respiratory action, and at this time, the temperature and ethylene concentration are increased by respiration.

Ethylene is a natural hormone released during storage of fruit vegetables, which increases the respiration of fruit vegetables, promotes overripening, and promotes spoilage. With regular plastic containers, the container breathability, ethylene gas removal, etc. are not properly controlled, making it impossible to maintain the freshness of fruit vegetables to result in the occurrence of a problem in that the storage period becomes very short due to this.

Further, in general, injection molded products using plastics use polyethylene or polypropylene-based plastic resins as a main raw material, and since the injection molded products, etc. do not have antibacterial properties against bacteria, mold, or the like, they are manufactured by adding plastic molding antibacterial agents containing silver with antibacterial properties in order to extend the shelf life. However, there has been a problem in that the manufacturing cost increased when using such antibacterial agents. Eventually, research was needed on food containers that could improve antibacterial properties at low cost, improve breathability inside the containers, and remove gas generated inside the containers.

### [Disclosure]

### [Technical Problem]

The technical problem to be achieved by the present disclosure is to provide a composition for a food container, which can maintain carbon dioxide and ethylene gas at an appropriate concentration inside a container containing fruit vegetables and/or fruits and improve antibacterial properties, and a method for manufacturing a food container using the same.

However, the problem to be solved by the present disclosure is not limited to the problem mentioned above, and other problems not mentioned will be clearly understood by those skilled in the art from the following description.

### [Technical Solution]

One embodiment of the present disclosure provides a composition for a food container, including a polymer resin, zeolite, an antibacterial additive, and a porous adsorption powder.

According to one embodiment of the present disclosure, zeolite contains pores, and the pores may have an average size of 300 mesh or more to 500 mesh or less.

According to one embodiment of the present disclosure, the antibacterial additive may be Hinokitiol.

According to one embodiment of the present disclosure, the porous adsorption powder may have an average particle size of 500 mesh or more to 700 mesh or less.

According to one embodiment of the present disclosure, the porous adsorption powder may include one or more selected from the group consisting of montmorillonite, bio-ceramic, pegmatite, fine silica powder, China clay, diatomaceous earth, bentonite, titanium dioxide, tourmaline, germanium, quartz porphyry, shell, pumice, vermiculite, sericite, quartz, muscovite, yellow ocher, kaolin, activated carbon, and combinations thereof.

According to one embodiment of the present disclosure, the polymer resin may include one or more selected from the group consisting of polypropylene, polyester, polyethylene, polystyrene, polylactic acid, polybutylene succinate, polyhydroxybutyrate, polyhydroxyalkanoate, poly(cyclohexane-1,4-dimethylene terephthalate), and combinations thereof.

According to one embodiment of the present disclosure, the polymer resin may be contained in an amount of 75 parts by weight or more to 98 parts by weight or less based on 100 parts by weight of the composition.

According to one embodiment of the present disclosure, zeolite may be contained in an amount of 1 part by weight or more to 10 parts by weight or less based on 100 parts by weight of the composition.

According to one embodiment of the present disclosure, the antibacterial additive may be contained in an amount of 0.1 part by weight or more to 5 parts by weight or less based on 100 parts by weight of the composition.

According to one embodiment of the present disclosure, the porous adsorption powder may be contained in an amount of 1 part by weight or more to 10 parts by weight or less based on 100 parts by weight of the composition.

One embodiment of the present disclosure provides a method for manufacturing a food container, the method including steps of: stirring the composition for a food container; vacuum-drying the stirred composition; and molding the vacuum-dried composition.

According to one embodiment of the present disclosure, the vacuum-drying step may be performed at 75°C or higher to 85°C or lower for 2 hours or more to 4 hours or less.

According to one embodiment of the present disclosure, the molding step may be performing molding into a pellet shape using a twin-screw extruder at a temperature of 180°C or higher to 245°C or lower.

According to one embodiment of the present disclosure, the method may further include a step of drying the molded composition after the molding step.

According to one embodiment of the present disclosure, the drying step may be performing drying in a vacuum oven at 100°C or higher to 120°C or lower for 6 hours or more to 8 hours or less.

### [Advantageous Effects]

The composition for a food container according to one embodiment of the present disclosure adsorbs and removes ethylene gas, carbonic acid gas, etc. generated by the respiratory action of fruit vegetables and fruits, and prevents spoilage caused by microorganisms with the antibacterial action, and thus can freshly keep fruit vegetables and fruits for a long period of time.

The method for manufacturing a food container according to the present disclosure can increase the adsorption efficiency of ethylene gas, carbonic acid gas, etc., and improve the antibacterial effect.

### [Description of Drawings]

FIG. 1 is a photograph of a food container according to one embodiment of the present disclosure.
FIG. 2 is a flowchart of a method for manufacturing a food container according to one embodiment of the present disclosure.
FIG. 3A is a photograph taken before storing a banana in the food container of Comparative Example 1, and FIG. 3B is a photograph taken after storing it under refrigeration at 15°C for 10 days.
FIG. 4A is a photograph taken before storing a banana in the food container of Example 1, and FIG. 4B is a photograph taken after storing it under refrigeration at 15°C for 10 days.
FIG. 5A is a photograph taken before storing a peach in the food container of Comparative Example 1, and FIG. 5B is a photograph taken after storing it at room temperature (23 ± 2°C) for 10 days.
FIG. 6A is a photograph taken before storing a peach in the food container of Example 1, and FIG. 6B is a photograph taken after storing it at room temperature (23 ± 2°C) for 10 days.
FIG. 7A is a photograph taken before storing apricots in the food container of Comparative Example 1, FIG. 7B is a photograph taken after storing them at room temperature (23 ± 2°C) for 10 days, and FIG. 7C is a photograph taken after storing them at room temperature (23 ± 2°C) for 14 days.
FIG. 8A is a photograph taken before storing apricots in the food container of Example 1, FIG. 8B is a photograph taken after storing them at room temperature (23 ± 2°C) for 10 days, and FIG. 8C is a photograph taken after storing them at room temperature (23 ± 2°C) for 14 days.
FIG. 9A is a photograph taken before storing lettuce in the food container of Example 1, and FIG. 9B is a photograph taken after storing it under refrigeration at 15°C for 31 days.

### [Mode for Disclosure]

In this specification, when a part "includes" a certain component, this means that it may further include other components rather than exclude other components, unless specifically stated to the contrary.

In this specification, "A and/or B" means "A and B, or A or B."

In this specification, the "mesh" unit may refer to the size of a particle diameter that can pass through the number of meshes included in a square with sides of 1 inch.

In this specification, "pore" may mean a space contained within a particle, and it may mean that the space is completely contained within the particle or partially exposed to communicate with the outside, and is exposed at both ends of the particle so that the particle has been penetrated.

The drawings attached to this specification illustrate preferred embodiments of the invention, and explain the principles of the present disclosure together with the description of the invention, and the scope of the invention is not limited thereto. Meanwhile, the shape, size, scale, ratio, or the like of elements in the drawings included in this specification may be exaggerated to emphasize a clearer description.

Hereinafter, the present disclosure will be described in more detail.

One embodiment of the present disclosure provides a composition for a food container, including a polymer resin, zeolite, an antibacterial additive, and a porous adsorption powder.

The composition for a food container according to one embodiment of the present disclosure adsorbs and removes ethylene gas, carbonic acid gas, etc. generated by the respiratory action of fruit vegetables and fruits, and prevents spoilage caused by microorganisms with the antibacterial action, and thus can freshly keep fruit vegetables and fruits for a long period of time.

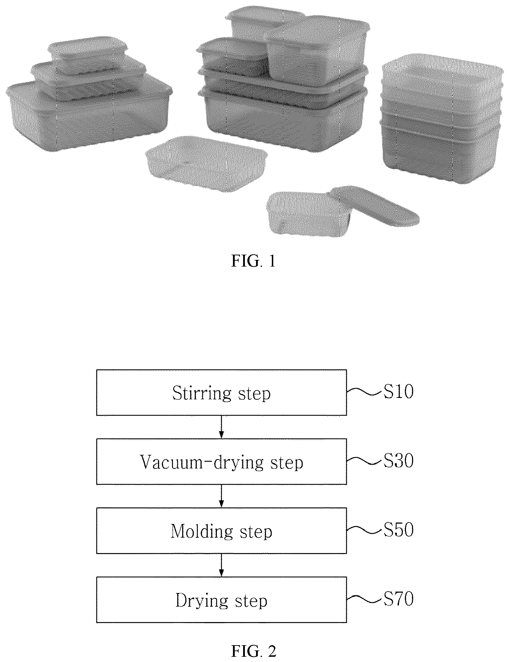

FIG. 1 is a photograph of a food container according to one embodiment of the present disclosure. The food container according to one embodiment of the present disclosure will be described in detail with reference to FIG. 1 above.

According to one embodiment of the present disclosure, zeolite contains pores, and the pores may have an average size of 300 mesh or more to 500 mesh or less. Specifically, the pores contained in zeolite may have an average size of 320 mesh or more to 480 mesh or less, 340 mesh or more to 460 mesh or less, 360 mesh or more to 440 mesh or less, or 380 mesh or more to 420 mesh or less. In this specification, "the size of the pores" may mean a diameter of the pores, and the diameter may mean the longest distance out of two points where a straight line passing through the inside of the pores inside meets. The performance of adsorbing a gas contained inside the container can be improved by adjusting the average size of the pores contained in zeolite within the above-described range.

According to one embodiment of the present disclosure, the antibacterial additive may be Hinokitiol. It is possible to improve the antibacterial properties of the container and reduce the manufacturing cost of the container at the same time by selecting Hinokitiol as the antibacterial additive as described above.

According to one embodiment of the present disclosure, the porous adsorption powder may have an average particle size of 500 mesh or more to 700 mesh or less. Specifically, the porous adsorption powder may have an average particle size of 520 mesh or more to 680 mesh or less, 540 mesh or more to 660 mesh or less, 560 mesh or more to 640 mesh or less, or 580 mesh or more to 620 mesh or less. In this specification, "the size of the particles" may mean a diameter of the particles, and the diameter may mean the longest distance out of two points where a straight line passing through the inside of the particles meets. The average particle size of the porous adsorption powder is adjusted as described above so that components included in the composition may be uniformly mixed to prevent the physical properties of the food container from being deteriorated, and improve the performance of adsorbing the gas contained inside the container.

According to one embodiment of the present disclosure, the porous adsorption powder may include one or more selected from the group consisting of montmorillonite, bio-ceramic, pegmatite, fine silica powder, China clay, diatomaceous earth, bentonite, titanium dioxide, tourmaline, germanium, quartz porphyry, shell, pumice, vermiculite, sericite, quartz, muscovite, yellow ocher, kaolin, activated carbon, and combinations thereof. The gas adsorption performance of the food container can be improved and deterioration of the physical properties of the food container can be prevented by selecting the porous adsorption powder from those described above.

According to one embodiment of the present disclosure, the polymer resin may include one or more selected from the group consisting of polypropylene (PP), polyester, polyethylene (PE), polystyrene (PS), polylactic acid (PLA), polybutylene succinate (PBS), polyhydroxybutyrate (PHB), polyhydroxyalkanoate (PHA), poly(cyclohexane-1,4-dimethylene terephthalate) (PCT), and combinations thereof. It is possible to secure the impact strength and durability of the food container and implement weight reduction at the same time by selecting the polymer resin from those described above.

According to one embodiment of the present disclosure, the polymer resin may be contained in an amount of 75 parts by weight or more to 98 parts by weight or less based on 100 parts by weight of the composition. Specifically, the polymer resin may be contained in an amount of 77 parts by weight or more to 96 parts by weight or less, 79 parts by weight or more to 94 parts by weight or less, 81 parts by weight or more to 92 parts by weight or less, or 83 parts by weight or more to 90 parts by weight or less based on 100 parts by weight of the composition. It is possible to secure the impact strength and durability of the food container and implement weight reduction at the same time by adjusting the content of the polymer resin within the above-described range.

According to one embodiment of the present disclosure, zeolite may be contained in an amount of 1 part by weight or more to 10 parts by weight or less based on 100 parts by weight of the composition. Specifically, zeolite may be contained in an amount of 3 parts by weight or more to 8 parts by weight or less or 5 parts by weight or more to 6 parts by weight or less based on 100 parts by weight of the composition. The performance of adsorbing the gas contained inside the container can be improved by adjusting the content of zeolite within the above-described range.

According to one embodiment of the present disclosure, the antibacterial additive may be contained in an amount of 0.1 part by weight or more to 5 parts by weight or less based on 100 parts by weight of the composition. Specifically, the antibacterial additive may be contained in an amount of 1 part by weight or more to 4 parts by weight or less or 2 parts by weight or more to 3 parts by weight or less based on 100 parts by weight of the composition. The antibacterial properties of the container can be improved by adjusting the content of the antibacterial additive within the above-described range.

According to one embodiment of the present disclosure, the porous adsorption powder may be contained in an amount of 1 part by weight or more to 10 parts by weight or less based on 100 parts by weight of the composition. Specifically, the porous adsorption powder may be contained in an amount of 3 parts by weight or more to 8 parts by weight or less or 5 parts by weight or more to 6 parts by weight or less based on 100 parts by weight of the composition. The performance of adsorbing the gas contained inside the container can be improved by adjusting the content of the porous adsorption powder within the above-described range.

One embodiment of the present disclosure provides a method for manufacturing a food container, the method including: a step S10 of stirring the composition for a food container; a step S30 of vacuum-drying the stirred composition; and a step S50 of molding the vacuum-dried composition.

The method for manufacturing a food container according to the present disclosure can increase the adsorption efficiency of ethylene gas, carbonic acid gas, etc., and improve the antibacterial effect.

FIG. 2 is a flowchart of a method for manufacturing a food container according to one embodiment of the present disclosure. The method for manufacturing a food container according to one embodiment of the present disclosure will be described in detail with reference to FIG. 2.

According to one embodiment of the present disclosure, the method for manufacturing a food container includes the step S10 of stirring the composition for a food container.

According to one embodiment of the present disclosure, the method for manufacturing a food container includes the step S30 of vacuum-drying the stirred composition. The composition can be uniformly mixed, and deterioration of the physical properties due to partial aggregation of specific components can be prevented by including the step S30 of vacuum-drying the stirred composition as described above.

According to one embodiment of the present disclosure, the method for manufacturing a food container includes the step S50 of molding the vacuum-dried composition. Deterioration of the physical properties due to an excessive amount of moisture contained in the food container can be prevented by including the step S50 of molding the vacuum-dried composition as described above.

According to one embodiment of the present disclosure, the vacuum-drying step S30 may be performed at 75°C or higher to 85°C or lower for 2 hours or more to 4 hours or less. Specifically, the vacuum-drying step S30 may have a temperature of 77°C or higher to 83°C or lower or 79°C or higher to 81°C or lower. Further, the vacuum-drying step S30 may be performed for a performance time of 1.5 hours or more to 3.5 hours or less. Moisture contained in the composition can be easily removed by adjusting the performance conditions of the vacuum-drying step as described above.

According to one embodiment of the present disclosure, the molding step S50 may be performing molding into a pellet shape using a twin-screw extruder at a temperature of 180°C or higher to 245°C or lower. Specifically, the temperature of the molding step S50 may be performing molding with a twin-screw extruder at a temperature of 185°C or higher to 240°C or lower, 190°C or higher to 235°C or lower, 195°C or higher to 230°C or lower, or 200°C or higher to 220°C or lower. The polymer resin can be molded into a pellet shape without denaturation by adjusting the conditions of the molding step S50 as described above.

According to one embodiment of the present disclosure, a step S70 of drying the molded composition may be further included after the molding step S50. Impurities included in the molding process can be removed by further including the step S70 of drying the molded composition after the molding step S50 as described above.

According to one embodiment of the present disclosure, the drying step S70 may be performing drying in a vacuum oven at 100°C or higher to 120°C or lower for 6 hours or more to 8 hours or less. Specifically, the drying step S70 may have a temperature of 100°C or higher to 120°C or lower, 102°C or higher to 118°C or lower, 104°C or higher to 116°C or lower, 106°C or higher to 114°C or lower, or 108°C or higher to 112°C or lower, and the drying step S70 may be performed in a vacuum oven for performance time of a 6.5 hours or more to 7.5 hours or less. The efficiency of removing the impurities included in the molding process can be improved by adjusting the conditions of the drying step S70 as described above.

Hereinafter, the present disclosure will be described in detail with reference to Examples in order to describe the present disclosure specifically. However, the embodiments according to the present disclosure may be modified into various other forms, and the scope of the present disclosure should not be construed as being limited to the Examples described below. The Examples of this specification are provided to more completely describe the present disclosure to those skilled in the art.

### <Example 1>

A composition including 87% by weight of polyethylene (PE) resin as a polymer resin, 5% by weight of synthetic zeolite with a pore size of 400 mesh, 3% by weight of Hinokitiol as an antibacterial additive, and 5% by weight of diatomaceous earth with a particle size of 600 mesh as a porous adsorption powder was stirred and then vacuum-dried at 80°C for 3 hours. Afterwards, it was molded into a pellet shape using a twin-screw extruder at a temperature of 210°C, and dried in a vacuum oven at 110°C for 7 hours. Afterwards, it was manufactured into the shape of a container.

### <Comparative Example 1>

A food container made of polyethylene was manufactured.

### <Experimental Example 1: Freshness Experiment>

Banana, peach, apricots, and lettuce were contained in each food container of Comparative Example 1 and Example 1, and photos before storage were taken and photos after storage under specific conditions were taken to compare appearance states thereof.

Specifically, the states of bananas were compared and confirmed before and after being stored under refrigeration at 15°C for 10 days, the states of peaches were compared and confirmed before and after being stored at room temperature (23 ± 2°C) for 10 days, the states of apricots were compared and confirmed before and after being stored at room temperature (23 ± 2°C) for 10 days, and the states of the lettuce were compared and confirmed before and after being stored under refrigeration at 15°C for 31 days.

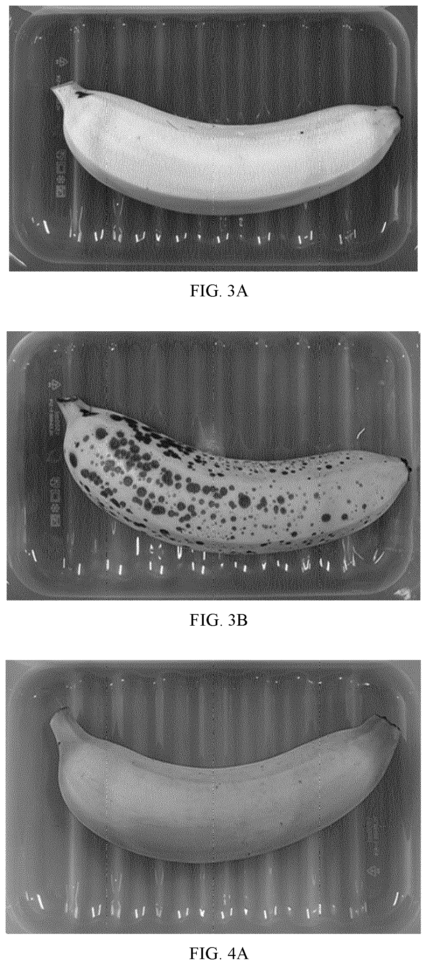

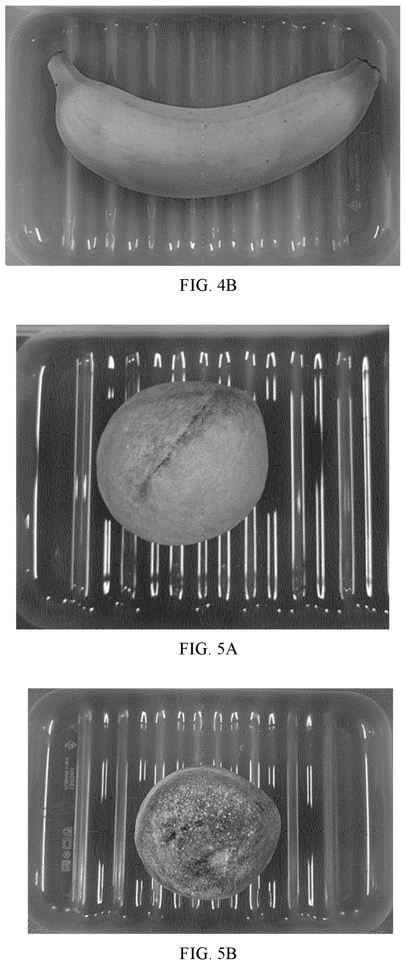

FIG. 3A is a photograph taken before storing a banana in the food container of Comparative Example 1, and FIG. 3B is a photograph taken after storing it under refrigeration at 15°C for 10 days. FIG. 4A is a photograph taken before storing a banana in the food container of Example 1, and FIG. 4B is a photograph taken after storing it under refrigeration at 15°C for 10 days. Referring to FIGS. 3A, 3B, 4A, and 4B, it was confirmed that the food container made of polyethylene of Comparative Example 1 had mostly black spots formed on the surface of the banana, but the food container of Example 1, which is one embodiment of the present disclosure, was confirmed to maintain the banana in its initial state.

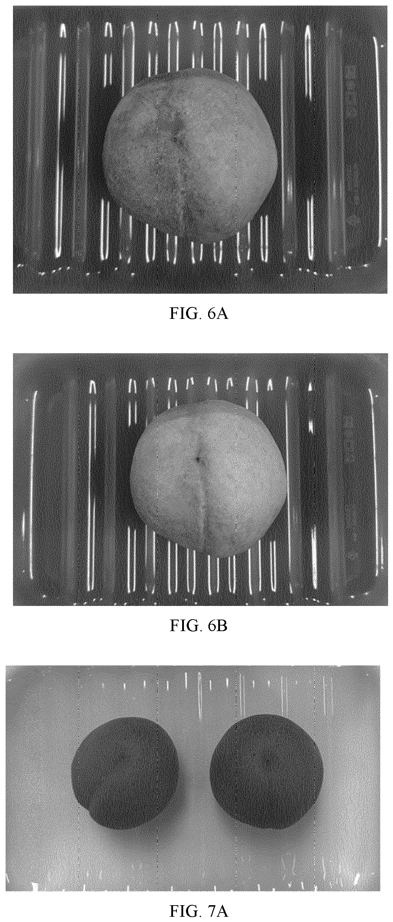

FIG. 5A is a photograph taken before storing a peach in the food container of Comparative Example 1, and FIG. 5B is a photograph taken after storing it at room temperature (23 ± 2°C) for 10 days. FIG. 6A is a photograph taken before storing a peach in the food container of Example 1, and FIG. 6B is a photograph taken after storing it at room temperature (23 ± 2°C) for 10 days. Referring to FIGS. 5A, 5B, 6A, and 6B, it was confirmed that the peach stored in the food container made of polyethylene of Comparative Example 1 was spoiled, but the peach stored in the food container of Example 1, which is one embodiment of the present disclosure, was confirmed to maintain its initial state.

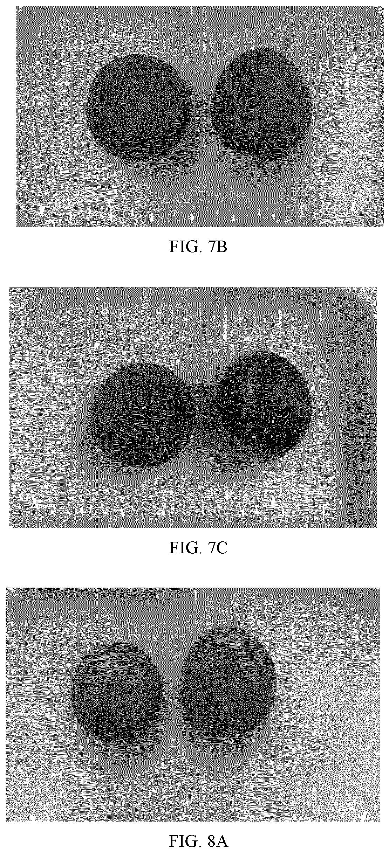

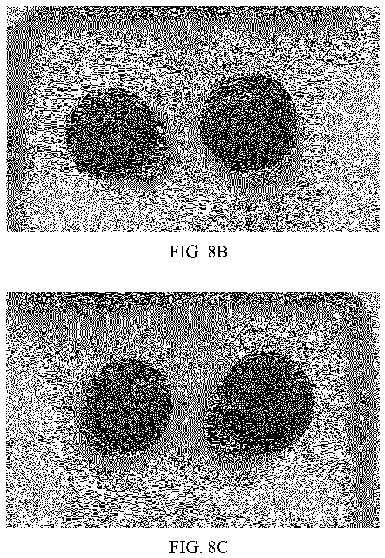

FIG. 7A is a photograph taken before storing apricots in the food container of Comparative Example 1, FIG. 7B is a photograph taken after storing them at room temperature (23 ± 2°C) for 10 days, and FIG. 7C is a photograph taken after storing them at room temperature (23 ± 2°C) for 14 days. FIG. 8A is a photograph taken before storing apricots in the food container of Example 1, FIG. 8B is a photograph taken after storing them at room temperature (23 ± 2°C) for 10 days, and FIG. 8C is a photograph taken after storing them at room temperature (23 ± 2°C) for 14 days. Referring to FIGS. 7A to 7C, and 8A to 8C, it was confirmed that the apricots stored in the food container made of polyethylene of Comparative Example 1 were spoiled, but the apricots stored in the food container of Example 1, which is one embodiment of the present disclosure, were confirmed to maintained their initial state.

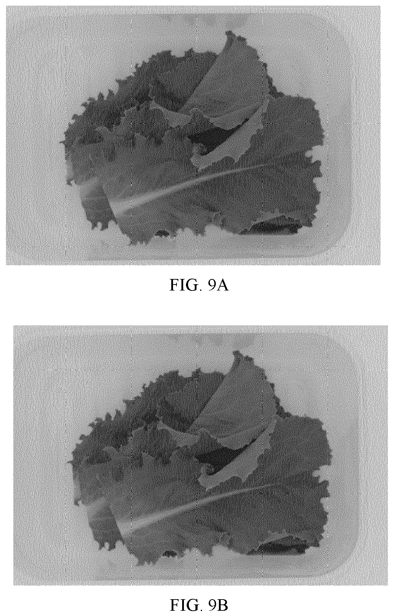

FIG. 9A is a photograph taken before storing lettuce in the food container of Example 1, and FIG. 9B is a photograph taken after storing it under refrigeration at 15°C for 31 days. Referring to FIG. 9, it was confirmed that the lettuce stored in the food container of Example 1, which is one embodiment of the present disclosure, maintained its initial state.

### <Experimental Example 2: Antibacterial Experiment>

An antibacterial experiment was performed on each of the food containers of Comparative Example 1 and Example 1 in accordance with JIS Z 2801:2010E. Specifically, tests were conducted on antibacterial properties against Escherichia coli, Staphylococcus aureus, Salmonella enterica, and Pseudomonas aeruginosa, and as a result, the values of an antibacterial rate and an eradication rate were summarized in Table 1 below. The antibacterial rate represents a ratio at which the growth of bacteria in the sample is inhibited compared to the control group by comparing the sample with a control group without antibacterial activity, and the eradication rate refers to a ratio of the number of bacteria remaining in the sample after sterilization treatment compared to the initial number of bacteria in the sample.

As a sample, the dish prepared in Example 1 was made into a film-type specimen with a width of 50 mm x a length of 50 mm and used, and as a control group, the dish of Comparative Example 1 was made into a specimen of the same shape and used. The test was conducted over three times (1st, 2nd, and 3rd).

Escherichia coli ATCC 8739, Staphylococcus aureus ATCC 6538p, Salmonella enterica KCTC2515, and Pseudomonas aeruginosa ATCC 9027 were used as test strains, respectively. Inoculation and culture of the test strains were carried out according to the pour plate method in accordance with JIS Z 2801:2010E.

The concentrations of the inoculated strains were 3.5 × 10⁵ CFU/film of Escherichia coli, 2.7 × 10⁵ CFU/film of Staphylococcus aureus, 3.8 × 10⁵ CFU/film of Salmonella enterica, and 3.5 × 10⁵ CFU/film of Pseudomonas aeruginosa. The culture medium used was 1/500 Nutrient broth (BD), inoculation was performed at 35°C for 24 hours, and culturing was performed at 35°C for 48 hours.

**[Table 1]**

| Test bacteria | Results | | | | | |
|---|---|---|---|---|---|---|
| | Concentration of inoculated bacteria (CFU/film) | Control group (CFU/film) | Sample (CFU/film) | | Antibacterial activity | |
| | | | | | Antibacterial rate (R, %) | Eradication rate (%) |
| Escherichia coli ATCC 8739 | 3.5 x 10⁵ | 3.0 x 10⁶ | 1st | <1 | 6.5 | >99.9% |
| | | | 2nd | <1 | 6.5 | >99.9% |
| | | | 3rd | <1 | 6.5 | >99.9% |
| Staphylococcus aureus ATCC 6538P | 2.7 x 10⁵ | 2.5 x 10⁶ | 1st | <1 | 6.4 | >99.9% |
| | | | 2nd | <1 | 6.4 | >99.9% |
| | | | 3rd | <1 | 6.4 | >99.9% |
| Salmonella enterica KCTC2515 | 3.8 x 10⁵ | 9.6 x 10⁶ | 1st | <1 | 7.0 | >99.9% |
| | | | 2nd | <1 | 7.0 | >99.9% |
| | | | 3rd | <1 | 7.0 | >99.9% |
| Pseudomonas aeruginosa ATCC 9027 | 3.5 x 10⁵ | 3.5 x 10⁶ | 1st | <1 | 6.9 | >99.9% |
| | | | 2nd | <1 | 6.9 | >99.9% |
| | | | 3rd | <1 | 6.9 | >99.9% |
| The antibacterial rate (%) calculates the percentage of bacterial growth inhibited compared to the control group by comparing the sample with a control group without antibacterial activity, and is calculated using the following Equation 1. | | | | | | |
| [Equation 1] | | | | | | |
| {(Number of bacteria in control group)-(Number of bacteria in sample)}/(Number of bacteria in control group)x100(%) | | | | | | |
| eradication rate (%) calculates the number of bacteria remaining in the sample 24 hours after the start of the test, and is calculated using Equation 2 below. | | | | | | |
| [Equation 2] | | | | | | |
| f (Initial number of bacteria)-(Number of bacteria 24 hours after start of test)}/(Initial number of bacteria)x100(%) | | | | | | |

As a result, since the antibacterial rates of the sample (Example 1) were small numerical values as 6.5%, 6.4%, 7.0%, and 6.9% as shown in Table 1, it was confirmed that the antibacterial activities were excellent compared to that of the control group (Comparative Example 1), and the eradication rates were also excellent as 99.9% or more.

### <Experimental Example 3: Anti-mold test>

Mold resistance values of the food container manufactured in Example 1 were respectively measured in accordance with the ASTM G21-15 test standard, and are summarized in Table 2 below.
1. Announced strains used: Aspergillus brasiliensis ATCC 9642, Chaetomium globosum ATCC 6205, Trichoderma virens ATCC 9645, Penicillium funiculosum ATCC 11797, Aureobasidium pullulans ATCC 15233
2. Culture conditions: (28 ~ 30) °C, 85% R.H. or higher, 28 days
3. Measurement grade: Grade 0: Does not grow/ Grade 1: Grows 10% or less on the food container/ Grade 2: Grows 10 to 30% or less on the food container/ Grade 3: Grows 30 to 60% or less on the food container/ Grade 4: Grows 60% or more on the food container

**[Table 2]**

| Measuring items | Experimental specifications | Experimental results |
|---|---|---|
| Grade | ASTM G21-15 | 0 |

Referring to Table 2, it was confirmed that the food container of Example 1 was rated Grade 0 and that mold did not grow.

Eventually, the composition for a food container, which is one embodiment of the present disclosure, and a method for manufacturing a food container using the same include a polymer resin, zeolite, an antibacterial additive, and a porous adsorption powder, thereby maintaining carbon dioxide and ethylene gas inside the container containing fruit vegetables and/or fruits at an appropriate concentration and enabling antibacterial properties to be improved.

### [Explanation of reference numerals]

S 10: Stirring step
S30: Vacuum-drying step
S50: Molding step
S70: Drying step

## Claims

1. A composition for a food container, comprising a polymer resin, zeolite, an antibacterial additive, and a porous adsorption powder.

2. The composition of claim 1, wherein zeolite contains pores, and the pores have an average size of 300 mesh or more to 500 mesh or less.

3. The composition of claim 1, wherein the antibacterial additive is Hinokitiol.

4. The composition of claim 1, wherein the porous adsorption powder has an average particle size of 500 mesh or more to 700 mesh or less.

5. The composition of claim 1, wherein the porous adsorption powder includes one or more selected from the group consisting of montmorillonite, bio-ceramic, pegmatite, fine silica powder, China clay, diatomaceous earth, bentonite, titanium dioxide, tourmaline, germanium, quartz porphyry, shell, pumice, vermiculite, sericite, quartz, muscovite, yellow ocher, kaolin, activated carbon, and combinations thereof.

6. The composition of claim 1, wherein the polymer resin includes one or more selected from the group consisting of polypropylene, polyester, polyethylene, polystyrene, polylactic acid, polybutylene succinate, polyhydroxybutyrate, polyhydroxyalkanoate, poly(cyclohexane-1,4-dimethylene terephthalate), and combinations thereof.

7. The composition of claim 1, wherein the polymer resin is contained in an amount of 75 parts by weight or more to 98 parts by weight or less based on 100 parts by weight of the composition.

8. The composition of claim 1, wherein zeolite is contained in an amount of 1 part by weight or more to 10 parts by weight or less based on 100 parts by weight of the composition.

9. The composition of claim 1, wherein the antibacterial additive is contained in an amount of 0.1 part by weight or more to 5 parts by weight or less based on 100 parts by weight of the composition.

10. The composition of claim 1, wherein the porous adsorption powder is contained in an amount of 1 part by weight or more to 10 parts by weight or less based on 100 parts by weight of the composition.

11. A method for manufacturing a food container, the method comprising steps of:
stirring the composition for a food container of any one or more of claims 1 to 10;
vacuum-drying the stirred composition; and
molding the vacuum-dried composition.

12. The method of claim 11, wherein the vacuum-drying step is performed at 75°C or higher to 85°C or lower for 2 hours or more to 4 hours or less.

13. The method of claim 11, wherein the molding step is performing molding into a pellet shape using a twin-screw extruder at a temperature of 180°C or higher to 245°C or lower.

14. The method of claim 11, further comprising a step of drying the molded composition after the molding step.

15. The method of claim 14, wherein the drying step is performing drying in a vacuum oven at 100°C or higher to 120°C or lower for 6 hours or more to 8 hours or less.
